(19) Europäisches Patentamt / European Patent Office / Office européen des brevets

(11) **EP 4 145 360 A1**

(12) **EUROPEAN PATENT APPLICATION**
published in accordance with Art. 153(4) EPC

(43) Date of publication:
**08.03.2023 Bulletin 2023/10**

(21) Application number: **21796215.8**

(22) Date of filing: **15.01.2021**

(51) International Patent Classification (IPC):
**G06N 20/00** (2019.01)

(52) Cooperative Patent Classification (CPC):
**G06N 20/00**

(86) International application number:
**PCT/JP2021/001234**

(87) International publication number:
**WO 2021/220556 (04.11.2021 Gazette 2021/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.04.2020 JP 2020078883
06.07.2020 JP 2020116497**

(71) Applicant: **Sony Group Corporation**
**Minato-Ku, Tokyo, 108-0075 (JP)**

(72) Inventors:
- **TAKI Yuko**
  **Tokyo 108-0075 (JP)**
- **TAKATSUKA Susumu**
  **Tokyo 108-0075 (JP)**
- **TETSUKAWA Hiroki**
  **Tokyo 108-0075 (JP)**

(74) Representative: **D Young & Co LLP**
**120 Holborn**
**London EC1N 2DY (GB)**

(54) **MACHINE LEARNING SYSTEM, MACHINE LEARNING DEVICE, AND MACHINE LEARNING METHOD**

(57) The present technology is to provide a machine learning system, a machine learning device and a machine learning method, which respectively prompts a person to engage in a target behavior with machine learning on a correlation between such a person's behavior and an environment around them.

The present technology is to provide a machine learning system including at least: a state acquisition unit that acquires at least state information regarding a behavior of a person; an evaluation unit that obtains a value function by evaluating environment information regarding an environment around the person at the time of acquiring the state information and the state information; and a machine learning classifier that performs reinforcement learning on the value function in order to prompt the person to engage in a target behavior and selects the environment information when the value function becomes highest.

FIG. 1

EP 4 145 360 A1

**Description**

TECHNICAL FIELD

The present technology relates to a machine learning system, a machine learning device and a machine learning method.

BACKGROUND ART

A technique has been used in which a computer performs machine learning of information regarding a behavior of a person in order to prompt the person to engage in a target behavior.

For example, Patent Document 1 discloses a "sales promotion system for providing a consumer with sales promotion information to induce consumption and promote sales using a computer network". Patent Literature 1 describes machine learning which will be performed on the basis of a behavior of the consumer after the sales promotion information is provided.

CITATION LIST

PATENT DOCUMENT

Patent Document 1: Japanese Patent Application Laid-Open No. 2019 028899

SUMMARY OF THE INVENTION

PROBLEMS TO BE SOLVED BY THE INVENTION

Conventionally, direct representation of information has been provided to a person, such as sales promotion information as described in Patent Document 1, in order to prompt the person to engage in a target behavior.

However, when a person changes their behavior, they may change it merely in response to a change in an environment around them without thinking.

Accordingly, the present technology is mainly intended to provide a machine learning system, a machine learning device and a machine learning method, which respectively prompt a person to engage in a target behavior with machine learning on a correlation between such a person's behavior and an environment around them.

SOLUTIONS TO PROBLEMS

The present technology is to provide a machine learning system including at least: a state acquisition unit that acquires at least state information regarding a behavior of a person; an evaluation unit that obtains a value function by evaluating environment information regarding an environment around the person at the time of acquiring the state information and the state information; and a machine learning classifier that performs reinforcement learning on the value function and selects the environment information when the value function becomes highest in order to prompt the person to engage in a target behavior.

The evaluation unit may be configured to calculate a reward on the basis of a difference between the state information and target state information regarding the target behavior, and to calculate the value function on the basis of the reward, the environment information and the state information.

The machine learning system may hold target state-related information including a plurality of pieces of target behavior information.

The target state-related information may include time-specific target state information and/or stage-specific target state information.

The environment information may include information regarding scents, lighting, temperature, humidity, video or sound.

The machine learning system may further include a scent control unit, and the scent control unit may be configured to control generated scent on the basis of the environment information selected by the machine learning classifier.

The machine learning system may further include an aromatization unit, and the aromatization unit may be configured to make items have scent on the basis of the environment information selected by the machine learning classifier, and

the machine learning classifier may determine which of the scent control unit and the aromatization unit will generate scent on the basis of the environment information.

The machine learning system may further include a lighting control unit, and the lighting control unit may be configured to control light to be emitted on the basis of the environment information selected by the machine learning classifier.

The machine learning system may further include an air conditioning unit, and the air conditioning unit may be configured to control a temperature and/or humidity on the basis of the environment information selected by the machine learning classifier.

The machine learning system may further include video control unit, and the video control unit may be configured to control a video to be displayed on the basis of the environment information selected by the machine learning classifier.

The machine learning system may further include sound control unit, and the sound control unit may be configured to control a sound to be played on the basis of the environment information selected by the machine learning classifier.

The value function may be divided into a plurality of value groups, and
the machine learning classifier may use the value function held by each of the plurality of value groups.

The machine learning system may further include a plurality of state acquisition units; and an achievement difficulty level calculation unit, and the achievement difficulty level calculation unit may be configured to calculate an achievement difficulty level for the target behavior on the basis of the state information acquired by each of the plurality of state acquisition units.

The achievement difficulty level may include an achievement rate indicating a degree to which the target behavior is prompted.

The achievement difficulty level may include a standard achievement time indicating a standard time for which the target behavior is prompted.

The achievement difficulty level may include a number of key variables indicating an average number of items in the environment information when the target behavior is prompted.

Further, the present technology is also to provide a machine learning device, including at least: a state acquisition unit configured to acquire at least state information regarding a behavior of a person; an evaluation unit configured to obtain a value function by evaluating the state information and environment information regarding an environment around the person when acquiring the state information; and a machine learning classifier that performs reinforcement learning on the value function and selects the environment information when the value function is the highest in order to prompt the person to engage in a target behavior.

Further, the present technology is also to provide a machine learning method, including at least: acquiring at least state information regarding a behavior of a person; obtaining a value function by evaluating the state information and environment information regarding an environment around the person when acquiring the state information; and performing reinforcement learning on the value function and selecting the environment information when the value function is the highest in order to prompt the person to engage in a target behavior.

BRIEF DESCRIPTION OF DRAWINGS

Fig. 1 is a block diagram illustrating a configuration of a machine learning system 1 according to one embodiment of the present technology.
Fig. 2 is a conceptual diagram illustrating one example of behavior modification according to one embodiment of the present technology.
Fig. 3 is a conceptual diagram illustrating one example of behavior modification according to one embodiment of the present technology.
Fig. 4 is a conceptual diagram illustrating one example of behavior modification according to one embodiment of the present technology.
Fig. 5 is a conceptual diagram illustrating one example of behavior modification according to one embodiment of the present technology.
Fig. 6 is a conceptual diagram illustrating one example of behavior modification according to one embodiment of the present technology.
Fig. 7 is a conceptual diagram illustrating one example of behavior modification according to one embodiment of the present technology.
Fig. 8 is a conceptual diagram illustrating one example of behavior modification according to one embodiment of the present technology.
Fig. 9 is a database illustrating one example of target state-related information according to one embodiment of the present technology.

Fig. 10 is a block diagram illustrating a hardware configuration of a machine learning device 10 according to one embodiment of the present technology.

Fig. 11 is a block diagram illustrating a configuration of a machine learning system 1 according to one embodiment of the present technology.

Fig. 12 is a block diagram illustrating a configuration of a scent control unit 23 according to one embodiment of the present technology.

Fig. 13 is a block diagram illustrating a configuration of a machine learning system 1 according to one embodiment of the present technology.

Fig. 14 is a flowchart illustrating a procedure of the machine learning system 1 according to one embodiment of the present technology.

Fig. 15 is a block diagram illustrating a configuration of a lighting control unit 24 according to one embodiment of the present technology.

Fig. 16 is a block diagram illustrating a configuration of an air conditioning unit 25 according to one embodiment of the present technology.

Fig. 17 is a block diagram illustrating a configuration of a video control unit 26 according to one embodiment of the present technology.

Fig. 18 is a block diagram illustrating a configuration of a sound control unit 27 according to one embodiment of the present technology.

Fig. 19 illustrates one example of a database used by the machine learning device 10 according to one embodiment of the present technology.

Fig. 20 is a flowchart illustrating an exemplified procedure of the machine learning device 10 according to one embodiment of the present technology.

Fig. 21 is a block diagram illustrating a configuration of a machine learning system 1 according to one embodiment of the present technology.

Fig. 22 is a diagram illustrating an achievement difficulty level calculated by an achievement difficulty level calculation unit 54 according to one embodiment of the present technology.

Fig. 23 is a diagram illustrating an achievement difficulty level calculated by the achievement difficulty level calculation unit 54 according to one embodiment of the present technology.

Fig. 24 is a flowchart illustrating a procedure of a machine learning method according to one embodiment of the present technology.

MODE FOR CARRYING OUT THE INVENTION

Preferred embodiments for carrying out the present technology will be described hereinbelow. Embodiments described below respectively illustrate an example of a representative embodiment of the present technology, and the scope of the present technology is not limited thereto. Further, each drawing is a schematic view, and is not necessarily exactly illustrated.

The present technology will be described in the following order.

1. First Embodiment of the Present Technology (Example 1 of Machine Learning System)

    (1) Overview
    (2) Evaluation Unit
    (3) Machine Learning Classifier
    (4) Flow of Behavior Modification
    (5) Pieces of Target State Information
    (6) Hardware Configuration

2. Second Embodiment of the Present Technology (Example 2 of Machine Learning System)

    (1) Overview
    (2) Scent Control Unit
    (3) Aromatization Unit
    (4) Lighting Control Unit
    (5) Air Conditioning Unit
    (6) Video Control Unit
    (7) Sound Control Unit

3. Third Embodiment of the Present Technology (Example 3 of Machine Learning System)
4. Fourth Embodiment of the Present Technology (Example 4 of Machine Learning System)
5. Fifth Embodiment of the Present Technology (Example 5 of Machine Learning System)

    (1) Overview
    (2) Achievement Difficulty Level

6. Sixth Embodiment of the Present Technology (Machine Learning Method)

[1. First Embodiment of the Present Technology (Example 1 of Machine Learning System)]

[(1) Overview]

A machine learning system according to one embodiment of the present technology can acquire a correlation between a behavior and an environment by evaluating and performing machine learning on information regarding a person's behavior and information regarding environment around such a person. Consequently, it is possible to prompt the person to engage in a target behavior by controlling the environment.

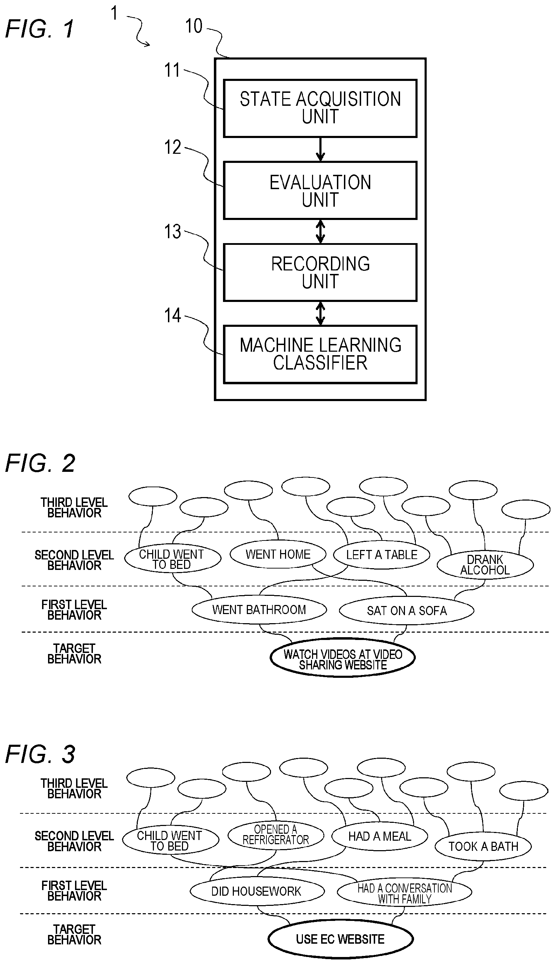

A configuration of the machine learning system according to one embodiment of the present technology will be described referring to Fig. 1. Fig. 1 is a block diagram illustrating a configuration of a machine learning system 1 according to one embodiment of the present technology.

As illustrated in Fig. 1, the machine learning system 1 can include, for example, a machine learning device 10. The machine learning device 10 can include, for example, a state acquisition unit 11, an evaluation unit 12, a recording unit 13, and a machine learning classifier 14.

The state acquisition unit 11 acquires at least state information regarding a person's behavior. Accordingly, the machine learning system 1 can identify how a person changes their behavior in response to a change in an environment.

Examples of the state information include cookies used to identify a user as they access a website, electronic commerce (e-commerce) purchase history, location information acquired by, for example, GPS (Global Positioning System), chat dialogue history, and other information acquired by sensing technologies.

Further, the state information may include information regarding the weather or temperature in an area where the person is present. In such a case, the machine learning system 1 can learn unique behavior modification specific to the weather or temperature in the area where the person is present.

The evaluation unit 12 obtains a value function by evaluating the state information and environment information regarding an environment around the person when acquiring the state information. Accordingly, a correlation between the environment information and the state information is acquired. Specific evaluation process will be described later.

Examples of the environment information include information regarding scent, lighting, temperature, humidity, video or sound. A specific example of the environment information will be described later.

The recording unit 13 records, for example, the state information and the environment information. Further, the machine learning system 1 acquires the state information but uses the environment information recorded without having been acquired.

The machine learning classifier 14 performs reinforcement learning on the value function and selects the environment information when the value function is the highest in order to prompt the person to engage in a target behavior. Accordingly, the correlation between the behavior and the environment is acquired.

A method of machine learning is not particularly limited, but for example, reinforcement learning can be used. Reinforcement learning is a machine learning training method in which software is able to perceive and interpret a current state (the state information in the present technology), and to determine a behavior that an agent should engage in (change in the environment information in the present technology). The agent (the machine learning classifier 14 in the present technology) can determine a behavior when a value is the highest with reinforcement learning by trial-and-error.

Examples of a conventional method for implementing reinforcement learning include Monte Carlo learning, dynamic programming, state-behavior-reward-state-behavior (SARSA) and Q-learning. The present technology will be described referring to Q-learning that is an example of reinforcement learning. Further, reinforcement learning algorithms other than Q-learning may be used in the present technology.

Furthermore, although not shown, the machine learning device 10 may be provided with a control unit that controls each component, a communication interface that establishes communication via a network, and the like.

[(2) Evaluation Unit]

As stated above, the evaluation unit 12 obtains a value function by evaluating the state information and the environment information regarding the environment around the person when acquiring the state information.

Although implementation of the evaluation unit 12 is not particularly limited, the evaluation unit 12 may be provided with a reward calculation unit (not shown) and a value calculation unit (not shown).

The state information regarding the person's behavior may change according to a change in the environment information. The reward calculation unit calculates a reward R on the basis of a difference between target state information regarding a target behavior and the state information when the machine learning system 1 changes the environment information. A larger value of the reward R indicates a smaller difference between the target state information and the state information. That is, the larger the value of the reward R is, the closer the person's behavior is to the target behavior.

The reward R can be expressed by, for example, the following Equation (1) using a score Pt according to the target state information and a score Pm according to the state information.

[Math. 1]

$$R = 1 - \frac{|Pt - Pm|}{Pt} \quad \cdots \quad (1)$$

A specific example will be described hereinbelow. "Purchasing a product A using an e-commerce website" is set as a target behavior. Then, 5 points are given when the person engages in a target behavior as the environment information changes.

Additionally, two points are given when the person engages in a behavior that is close to the target behavior, e.g. "access a website including the product A" as the environment information changes.

Fitting the numbers to Equation (1), the score Pt according to the target state information is "5". The score Pm according to the state information when the person engages in the target behavior as the environment information changes is also "5". At this time, the reward R is "1".

The score Pm according to the state information when the person engages in a behavior close to the target behavior as the environment information changes is "2". At this time, the reward R is "0.4".

The score Pm according to the state information when the person engages in a behavior other than these two behaviors as the environment information changes is "0". At this time, the reward R is also "0".

In other words, a value of the reward R increases as the person's behavior due to the change in the environment information is closer to the target behavior. The reward calculation unit calculates the highest reward R when the environment information changes.

The value calculation unit calculates a value function Q on the basis of the reward R, the environment information, and the state information. The value calculation unit calculates the value function Q on the basis of the state information when the environment information changes with the highest reward R. For example, a value function when the change at of the environment information is carried out for state information st at a time t is denoted by $Q(s_t, a_t)$.

The value function Q may be recorded by, for example, the recording unit 13. More specifically, the recording unit 13 may record the value function Q on a table for each state information or environment information.

[(3) Machine Learning Classifier]

As stated above, the machine learning classifier 14 performs reinforcement learning on the value function Q and selects the environment information when the value function Q is the highest.

This reinforcement learning will be described hereinbelow. The machine learning classifier 14 automatically learns by trial-and-error such that the value function Q becomes the highest. As the value function Q is higher, the person's behavior is closer to the target behavior. By performing reinforcement learning such that the value function Q becomes the highest, the machine learning classifier 14 can prompt the person to engage in the target behavior for the current behavior.

The machine learning classifier 14 updates the value function Q as the environment information is selected when the value function Q is the highest. For example, when the change at of the environment information is performed on the state information st at the time t and transition is made to the state information st+1 at the time t+1, the value function Q(st,at) is updated with the following Equation (2).

[Math. 2]

$$Q(s_t, a_t) \leftarrow Q(s_t, a_t) + \alpha \left[ R_{t+1} + \gamma \max_a Q(s_{t+1}, a) - Q(s_t, a_t) \right] \quad \cdots \quad (2)$$

$\alpha$ represents a learning coefficient. The learning coefficient $\alpha$ has a value falling within a range of $0 < \alpha \leq 1$; the value most often used is about 0.1.

$R_t+_1$ represents a reward obtained by the transition of the state information.

$\gamma$ represents a discount rate. The discount rate $\gamma$ has a value falling within a range of $0 < \gamma \leq 1$; the value most often used is about 0.9 to 0.99.

$\max Q(s_{t+1}, a)$ represents a future ideal value function. $\max Q(S_{t+1}, a)$ is a value function when a behavior a with the highest value function Q is selected in a state $s_{t+i}$ at a time t+1. The value function $\max Q(s_{t+1}, a)$ is multiplied by the discount rate $\gamma$.

The machine learning classifier 14 keeps updating the value function Q using Equation (2) stated above, and selects the environment information when the value function Q is the highest. Accordingly, the machine learning classifier 14 can select the environment information that can prompt the person to engage in the target behavior.

[(4) Flow of Behavior Modification]

It is assumed that a plurality of behavior modification is experienced before reaching the target behavior. This will be described with reference to Fig. 2. Fig. 2 is a conceptual diagram illustrating one example of behavior modification according to the present embodiment. As shown in Fig. 2, "watch videos at online streaming platform" is set as the target behavior. Then, a plurality of flows of behavior modification is set for prompting the person to engage in the target behavior. The machine learning classifier 14 can configure this flow of behavior modification with repeated reinforcement learning. The machine learning classifier 14 can prompt the person to engage in the target behavior by following this flow.

Additionally, behaviors of the person are categorized into a plurality of levels according to how much close they are to the target behavior. For example, a first level behavior may be a behavior closest to the target behavior. A second level behavior may be a behavior next closest to the target behavior. A value function Q related to the first level behavior is higher than a value function Q related to the second level behavior.

In this example, the first level behaviors include "went bathroom" and "sat on a sofa". The second level behaviors include "child went to bed", "went home", "left a table" and "drank alcohol". Then, a flow of behavior modification is configured by connecting each of a plurality of behaviors. For example, the characteristics of behavior modification for this individual demonstrate that they tends to engage in a behavior "went bathroom" in when "child went to bed" happens.

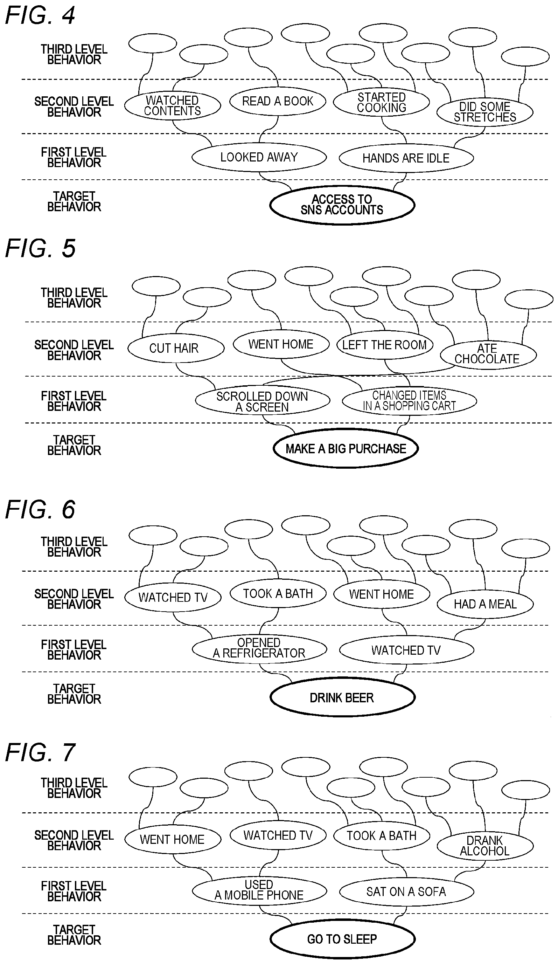

Other exemplified flows of behavior modification are illustrated in Figs. 3 to 7. Figs. 3 to 7 are each a conceptual diagram illustrating one example of behavior modification according to the present embodiment. Fig. 3 illustrates one example of a flow of behavior modification for prompt a person to engage in a target behavior, i.e. "use e-commerce website". Fig. 4 illustrates one example of a flow of behavior modification for prompt a person to engage in a target behavior, i.e. "access SNS (social networking service) accounts". Fig. 5 illustrates one example of a flow of behavior modification for prompt a person to engage in a target behavior, i.e. "make a big purchase". Fig. 6 illustrates one example of a flow of behavior modification for prompt a person to engage in a target behavior, i.e. "drink beer". Fig. 7 illustrates one example of a flow of behavior modification for prompt a person to engage in a target behavior, i.e. "go to sleep". As illustrated above, various flows of behavior modification can be configured according to a target behavior.

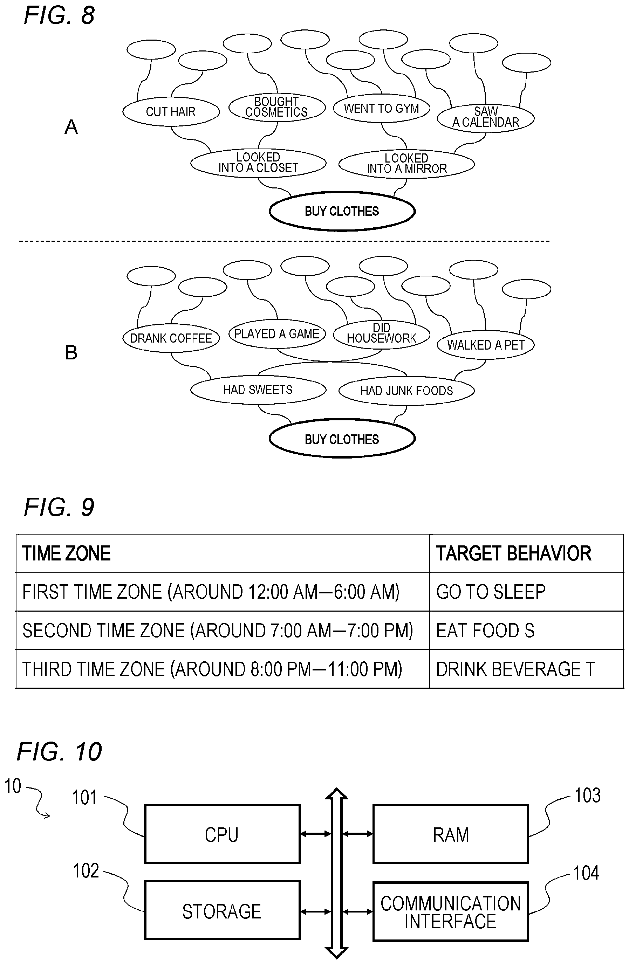

Further, even if the target behavior is the same, a flow of behavior modification for prompting a person to engage in the target behavior may be different depending on individuals. This will be described with reference to Fig. 8. Fig. 8 is a conceptual diagram illustrating one example of behavior modification according to the present embodiment. Fig. 8A illustrates one example of behavior modification of a certain individual. As shown in Fig. 8A, "buy clothes" is set as the target behavior.

On the other hand, Fig. 8B illustrates one example of behavior modification for an individual other than the individual illustrated in Fig. 8A. As shown in Fig. 8B, the target behavior is the same as Fig. 8A, but a flow of behavior modification for prompting a person to engage in the target behavior is different.

[(5) Pieces of Target State Information]

The machine learning device 10 according to one embodiment of the present technology may hold target state information regarding one target behavior, but may hold a plurality of pieces of target state information regarding a plurality of target behaviors. A part or all of the plurality of target behaviors can be set, for example, by time and/or by stage.

A part or all of the plurality of target behaviors can be set, for example, by time. More specifically, a part or all of the plurality of target behaviors can be categorized into, for example, a target behavior in a first time zone (for example, from 12:00 AM to 6:00 AM), a target behavior in a second time zone (for example, from 7:00 AM to 7:00 PM), and a target behavior in a third time zone (for example, from 8:00 PM to 11:00 PM) in a day.

The target behavior in the first time zone (for example, from 12:00 AM to 6:00 AM) may be, for example, "go to sleep". The target behavior in the second time zone (for example, from 7:00 AM to 7:00 PM) may be, for example, "eat food S". The target behavior in the third time zone (for example, from 8:00 PM to 11:00 PM) may be, for example, "drink beverage T".

A part or all of the plurality of target behaviors is set by time, whereby the target behavior can be flexibly set according to, for example, a time zone. For example, the machine learning device 10 can prompt a person to engage in the target behavior, i.e. "eat food S at 3:00 PM".

Alternatively, a part or all of the plurality of target behaviors can be set, for example, by stage. More specifically, a part or all of the plurality of target behaviors can be categorized into, for example, a target behavior in a first stage and a target behavior in a second stage. The target behavior in the first stage may be, for example, "go to shop U". The target behavior in the second stage may be, for example, "eat food S".

A part or all of the target behaviors are set by stage, whereby a plurality of target behaviors having a series of flows can be set. For example, the machine learning device 10 can prompt a person to engage in the target behavior, i.e. "go to shop U and eat food S".

Alternatively, a part or all of the plurality of target behaviors can be set, for example, by time and by stage. More specifically, it is possible to set the target behaviors in the first and second stages for the third time zone.

A part or all of the target behaviors are set by time and by stage, whereby a plurality of target behaviors having a series of flows can be set flexibly set according to, for example, a time zone. For example, the machine learning device 10 can prompt a person to engage in the target behavior, i.e. "go to shop U and eat food S in the morning".

To implement a scheme stated above, the machine learning device 10 according to one embodiment of the present technology may hold target state-related information including a plurality of pieces of target state information.

The target state-related information will be described with reference to Fig. 9. Fig. 9 is a database illustrating one example of target state-related information according to the present embodiment. As shown in Fig. 9, a time zone is associated with a target behavior. The target behavior in the first time zone (from 12:00 AM to 6:00 AM) is "go to sleep". The target behavior in the second time zone (from 7:00 AM to 7:00 PM) is "eat food S". The target behavior in the third time zone (from 8:00 PM to 11:00 PM) is, for example, "drink beverage T". In addition, the same applies to target state-related information set for each stage.

The target state-related information can be recorded in, for example, the recording unit 13 included in the machine learning device 10. Further, the target state-related information may be held by a computer device other than the machine learning device 10. For example, the target state-related information may be held in a server on cloud. In such a case, the machine learning device 10 may receive the target state-related information from the server via an information communication network.

[(6) Hardware Configuration]

A hardware configuration of the machine learning device 10 will be described with reference to Fig. 10. Fig. 10 is a block diagram illustrating a hardware configuration of the machine learning device 10 according to the present embodiment. As shown in Fig. 10, the machine learning device 10 can include a CPU 101, a storage 102, a random access memory (RAM) 103, and a communication interface 104 as components. The respective components are connected by, for example, a bus as a data transmission path.

The CPU 101 is implemented by, for example, a microcomputer, and controls each component of the machine learning device 10. The CPU 101 can function as, for example, the evaluation unit 12 or the machine learning classifier 14. The machine learning classifier 14 can be implemented by, for example, a program. The program can function by being read by the CPU 101.

The storage 102 stores control data such as programs and operation parameters used by the CPU 101. The storage 102 can be implemented using, for example, a hard disk drive (HDD) or a solid state drive (SSD). The storage 102 can function as, for example, the recording unit 13.

The RAM 103 temporarily stores, for example, a program executed by the CPU 101.

The communication interface 104 has a function of establishing communication via the information communication network using a communication protocol such as Wi-Fi, Bluetooth (registered trademark) or long term evolution (LTE).

The program that implements the machine learning classifier 14 and the like may be stored in a computer device or a computer system other than machine learning system 1. In this case, the machine learning system 1 can adopt a cloud service that provides functions of the program. Examples of the cloud service include software-as-a-service (SaaS), infrastructure-as-a-service (IaaS), and platform-as-a-service (PaaS).

Furthermore, the program can be stored using a variety of non-transitory computer-readable media and supplied to the computer. Non-transitory computer-readable media include a variety of tangible storage media. Examples of the non-transitory computer-readable medium include magnetic recording medium (e.g. flexible disk, magnetic tape or hard disk drive), magneto-optical recording medium (e.g. magneto-optical disk), compact disc read only memory (CD-ROM), CD-R, CD-R/W, and semiconductor memory (e.g. mask ROM, programmable ROM (PROM), erasable PROM (EPROM), flash ROM, or random access memory (RAM)). Furthermore, the program may be supplied to the computer by a variety of transitory computer-readable media. Examples of transitory computer-readable media include electrical signals, optical signals, and electromagnetic waves. The transitory computer-readable medium can supply the program to the computer via a wired communication path such as an electric wire and an optical fiber, or a wireless communication path.

[2. Second Embodiment of the Present Technology (Example 2 of Machine Learning System)]

[(1) Overview]

The machine learning system 1 according to one embodiment of the present technology may include an environmental control device for controlling an environment around a person. The environmental control device controls the environment around the person on the basis of the environment information selected by the machine learning classifier 14. Accordingly, the machine learning system 1 can prompt the person to engage in the target behavior.

The machine learning system 1 can promote, for example, sales by prompting a person to engage in a target behavior. The machine learning system 1 can control an internal or external environment of a shop in order to cause a customer to purchase a product. Additionally, a shop where the machine learning system 1 is employed is not limited to offline stores; it may be used in e-commerce website, i.e. online shopping malls. Alternatively, the machine learning system 1 is also used for websites or contents downloaded or streamed, and can promote access to such websites or contents.

Alternatively, the machine learning system 1 can improve, for example, a person's daily habits by prompting the person to engage in a target behavior. In particular, the machine learning system 1 can control an environment in order to prompt a person to quit smoking or drinking alcohol. Furthermore, the machine learning system 1 may help to overcome, for example, shopping addition, sleep deprivation, lack of exercise and the like.

Alternatively, the machine learning system 1 can cause a person to vote in elections by prompting the person to engage in a target behavior.

Alternatively, the machine learning system 1 can raise awareness of public health or moral awareness by prompting a person to engage in a target behavior. Specifically, the machine learning system 1 may be used to create awareness in waste management, to improve attitudes towards littering, rushing abroad and lining up, to follow recommendations such as covering coughs and keeping hands clean, and to raise awareness about bullying.

Alternatively, the machine learning system 1 can improve, for example, work efficiency by prompting a person to engage in a target behavior. More specifically, the machine learning system 1 can be used for improvement of concentration, learning to pay attention, and work-rest balance.

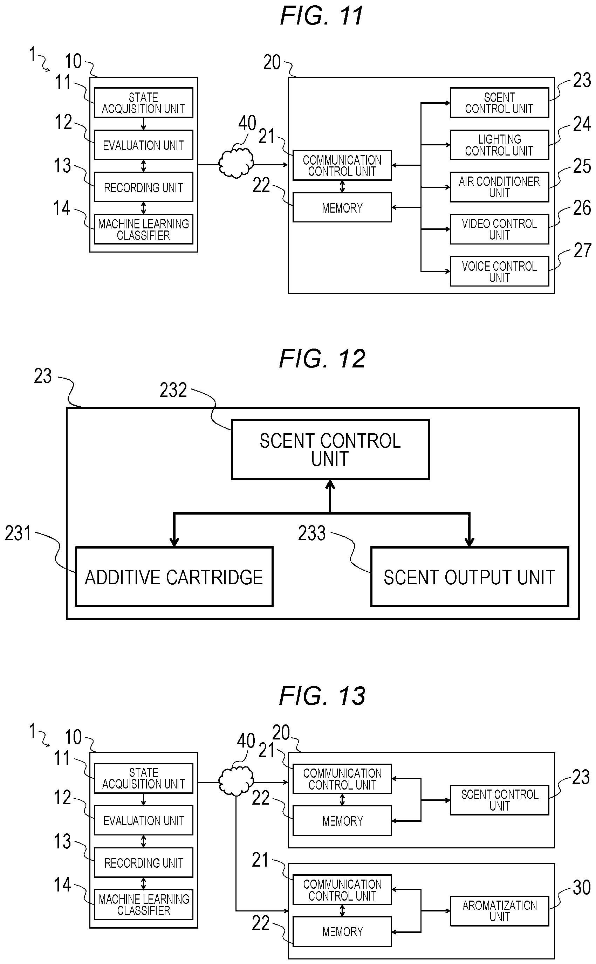

A configuration of the machine learning system 1 according to the present embodiment will be described referring to Fig. 11. Fig. 11 is a block diagram illustrating a configuration of the machine learning system 1 according to the present embodiment. Further, components similar to those in the first embodiment are denoted by the similar reference numerals, and detailed description thereof will be omitted.

As illustrated in Fig. 11, the machine learning system 1 according to the present embodiment may further include an environmental control device 20. The environmental control device 20 and the machine learning device 10 are connected via an information communication network 40. The environmental control device 20 controls an environment such as a scent, lighting, temperature, humidity, video or sound on the basis of the environment information selected by the machine learning classifier 14.

The environmental control device 20 can include, for example, a communication control unit 21, a memory 22, a scent control unit 23, a lighting control unit 24, an air conditioning unit 25, a video control unit 26 and a sound control unit 27.

Further, the environmental control device 20 may not have all of the scent control unit 23, the lighting control unit 24, the air conditioning unit 25, the video control unit 26 and the sound control unit 27; in other words, it may have at least one of these components.

In addition, the machine learning system 1 may include a plurality of environmental control devices 20. For example, when the machine learning system 1 includes two environmental control devices 20, one environmental control device 20 may include the scent control unit 23, and the other environmental control device 20 may include the lighting control unit 24.

The communication control unit 21 can communicate information with the machine learning device 10 via the information communication network 40. Furthermore, the communication control unit 21 may control each component.

The memory 22 can record information used by the environmental control device 20, for example, the environment information.

Note that the machine learning classifier 14 provided in the machine learning device 10 may be included in, for example, the environmental control device 20, or may be included in another computer device.

[(2) Scent Control Unit]

The environmental control device 20 can include, for example, the scent control unit 23 to control a scent around a person. The scent control unit 23 controls generated scent on the basis of the environment information selected by the machine learning classifier 14. The environmental control device 20 including the scent control unit 23 can be implemented using, for example, an aroma diffuser.

Further, the scent includes a scent that can be perceived by a person as a scent, as well as a scent that cannot be perceived by a person as a scent but is inhaled to exert some action on such a person. For example, inhaled sedatives or odorless gasses (e.g. oxygen or carbon dioxide) acting on the physical condition of a person by inhalation are also included in the scent.

The person is prompted to engage in the target behavior unconsciously by inhaling the scent optimized for them and controlled by the scent control unit 23.

A configuration of the scent control unit 23 will be described with reference to Fig. 12. Fig. 12 is a block diagram illustrating a configuration of the scent control unit 23 according to the present embodiment. As illustrated in Fig. 12, the scent control unit 23 can include, for example, an additive cartridge 231, a scent control unit 232 and a scent output unit 233.

The additive cartridge 231 is a component that stores additive for a scent. The additive cartridge 231 may be replaceable. The additive cartridge 231 may be, for example, a container such as a cylinder, a bottle, or a can containing the additive; a material such as paper, nonwoven fabric, or stone adsorbing the additive; or a solid body such as wax or soap mixed with the additive.

The additive may be, for example, a solid, a liquid or a gas including a powder and a gel, or a mixture thereof. The additive may be, for example, a naturally-derived fragrance, a synthetic fragrance obtained from chemical synthesis, or a prepared fragrance prepared by blending those fragrances. Alternatively, the additive may not contain fragrance.

The scent control unit 232 controls an additive for generating a scent on the basis of environment information. The scent control unit 232 can determine, for example, a ratio of each additive upon blending additives. Alternatively, the scent control unit 232 may determine a dilution rate. The ratio or the dilution rate is determined according to the environment information selected by the machine learning classifier 14.

Alternatively, the scent control unit 233 may control, for example, parameters to output the scent, such as a spray pressure and the number of sprays. The spray pressure or the number of sprays is determined according to the environment information selected by the machine learning classifier 14.

The scent output unit 233 outputs a scent on the basis of the information determined by the scent control unit 232.

Alternatively, the machine learning system 1 provided with the scent control unit 23 can prompt a person to engage in a target behavior by causing the person to inhale a specific scent. For example, the machine learning system 1 can generate a scent that induces a person physiologically to buy a specific product, whereby they order by mail or go to a shop to buy such a product.

Alternatively, a scent may be associated with specific contents. Therefore, the machine learning system 1 can cause a person to unconsciously learn association between the scent and the contents before performing reinforcement learning.

For example, the machine learning system 1 can cause a person to inhale a specific scent while watching a specific video. The video includes, for example, an advertisement related to a specific product. Therefore, the machine learning system 1 can cause the person to unconsciously learn association between the specific scent and the specific product. When the person inhales the specific scent from the machine learning system 1, it will induce the person to order it by mail or go to a shop to find it.

Alternatively, a scent may be associated with a specific environment. This environment is related to a place or an object that a person actually experiences. Examples of the place include shops, public transportations, movie theaters, theaters and theme parks. Therefore, the machine learning system 1 can cause a person to unconsciously learn association between the scent and the environment before performing reinforcement learning.

For example, the machine learning system 1 can cause a person to inhale a specific scent while they visit a specific shop. Therefore, the machine learning system 1 can cause the person to unconsciously learn association between the specific scent and the specific shop. When the person inhales the specific scent from the machine learning system 1 at a place different from the shop, it will induce the person to go to the shop or order a product displayed in the shop by mail.

For example, the machine learning system 1 can make a person inhale a scent generated from a specific product by an experience, such as drinking coffee. Therefore, the machine learning system 1 can cause the person to unconsciously learn association between the specific scent and the specific product. When the scent control unit 23 generates this specific scent, the machine learning system 1 will induce the person to order the specific product by mail or go to a shop to find it.

[(3) Aromatization Unit]

The scent may be adhered to a certain item. Examples of the item may include clothes, books, miscellaneous goods, promotional items or packing materials delivered to a person to be prompted to engage in a target behavior. The person is prompted to engage in the target behavior unconsciously by inhaling a scent which is adhered to the item and optimized for them.

To implement a scheme stated above, the machine learning system 1 can provide with an aromatization unit. This will be described with reference to Fig. 13. Fig. 13 is a block diagram illustrating a configuration of the machine learning system 1 according to the present embodiment. Further, components similar to those in the first embodiment are denoted by the similar reference numerals, and detailed description thereof will be omitted. As illustrated in Fig. 13, the machine learning system 1 further includes an aromatization unit 30. The aromatization unit 30 and the machine learning device 10 are connected via the information communication network 40. Furthermore, the aromatization unit 30 may have the similar configuration as the scent control unit 23.

The scent control unit 23 is disposed around a person to be prompted to engage in a target behavior. On the other hand, the aromatization unit 30 is disposed, for example, in a factory where the item is shipped. The aromatization unit 30 make the item have a scent on the basis of the environment information selected by the machine learning classifier 14.

The machine learning classifier 14 determines which of the scent control unit 23 and the aromatization unit 30 generates a scent on the basis of the environment information.

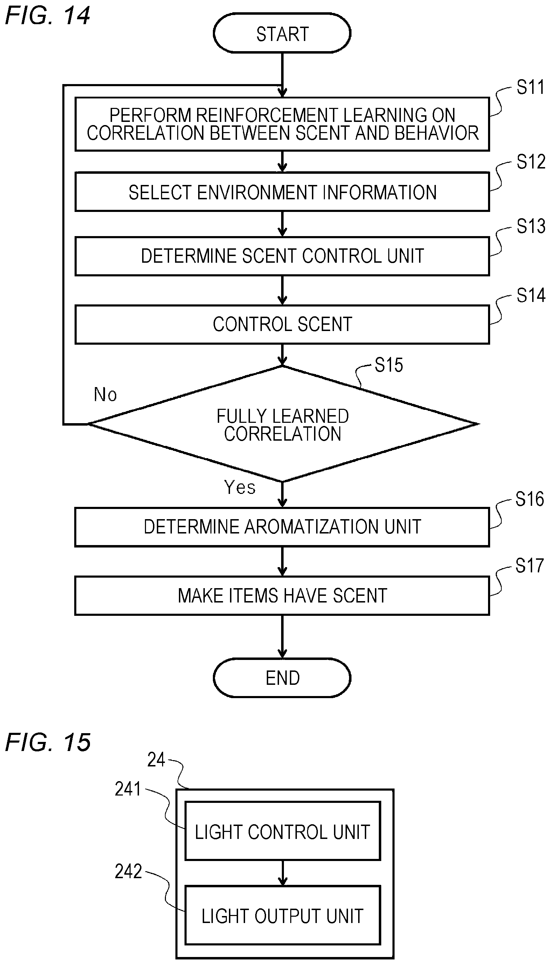

A procedure of the machine learning system 1 at this time will be described referring to Fig. 14. Fig. 14 is a flowchart illustrating a procedure of the machine learning system 1 according to the present embodiment.

As illustrated in Fig. 14, the machine learning classifier 14 performs reinforcement learning on a correlation between a scent and a behavior (step S11) in an initial stage of machine learning. The machine learning classifier 14 selects the environment information (step S12) and determines that the scent control unit 23 generates the scent (step S13). The scent control unit 23 controls the scent around a person (step S14). Steps S11 to S14 are repeated until the correlation between the scent and the behavior is sufficiently trained (step S15: NO).

At a stage where the correlation between the scent and the behavior has been sufficiently trained (step S15: YES), the machine learning classifier 14 determines that the aromatization unit 30 generates the scent (step S16), and the aromatization unit 30 makes an item have the scent (step S17).

Consequently, the machine learning system 1 can more flexibly control the scent around the person. For example, the machine learning classifier 14 performs reinforcement learning of the correlation between the scent and the behavior with high efficiency while the scent control unit 23 disposed around the person changes the scent in a short period of time in the initial stage of machine learning (for example, about 1 to 3 months from the start of learning). The machine learning classifier 14 determines a scent optimized for the target behavior.

Thereafter, the target behavior can be continuously prompted by, for example, delivery of items with the optimum scent for the target behavior. While the aromatization unit 30 changes the scent over a long period of time, the machine learning classifier 14 continues reinforcement learning of the correlation between the scent and the behavior.

[(4) Lighting Control Unit]

The description returns to Fig. 11. The environmental control device 20 can include, for example, the lighting control unit 24 to control lighting around a person. The lighting control unit 24 controls light to be emitted on the basis of the environment information selected by the machine learning classifier 14. The environmental control device 20 including the lighting control unit 24 can be implemented using, for example, a light bulb (including a so-called smart bulb).

The person is prompted to engage in the target behavior unconsciously by visually recognizing the light optimized for them and irradiated by the lighting control unit 24.

A configuration of the lighting control unit 24 will be described with reference to Fig. 15. Fig. 15 is a block diagram illustrating a configuration of the lighting control unit 24 according to the present embodiment. As illustrated in Fig. 15, the lighting control unit 24 can include, for example, a light control unit 241 and a light output unit 242.

The light control unit 241 controls a representation of light to be output. More specifically, the light control unit 241 can determine, for example, a color temperature and luminance of light. The color temperature or the luminance is determined according to the environment information selected by the machine learning classifier 14. For example, the color temperature may be determined to be 3500 to 3900 K, and the luminance may be determined to be 3000 to 4000 lm. Further, in a case where a range is determined as stated above, the light control unit 241 may randomly determine a value falling within this range. The machine learning device 10 can narrow this range upon repeated reinforcement learning. In addition, the same applies to other components described below.

The light output unit 242 outputs light on the basis of the information determined by the light control unit 241.

[(5) Air Conditioning Unit]

The environmental control device 20 can include, for example, the air conditioning unit 25 to control air around a person. The air conditioning unit 25 controls a temperature and/or humidity on the basis of the environment information selected by the machine learning classifier 14. The environmental control device 20 including the air conditioning unit 25 can be implemented using, for example, an air conditioner.

The person is prompted to engage in the target behavior unconsciously by the temperature and/or humidity optimized for them and controlled by the air conditioning unit 25.

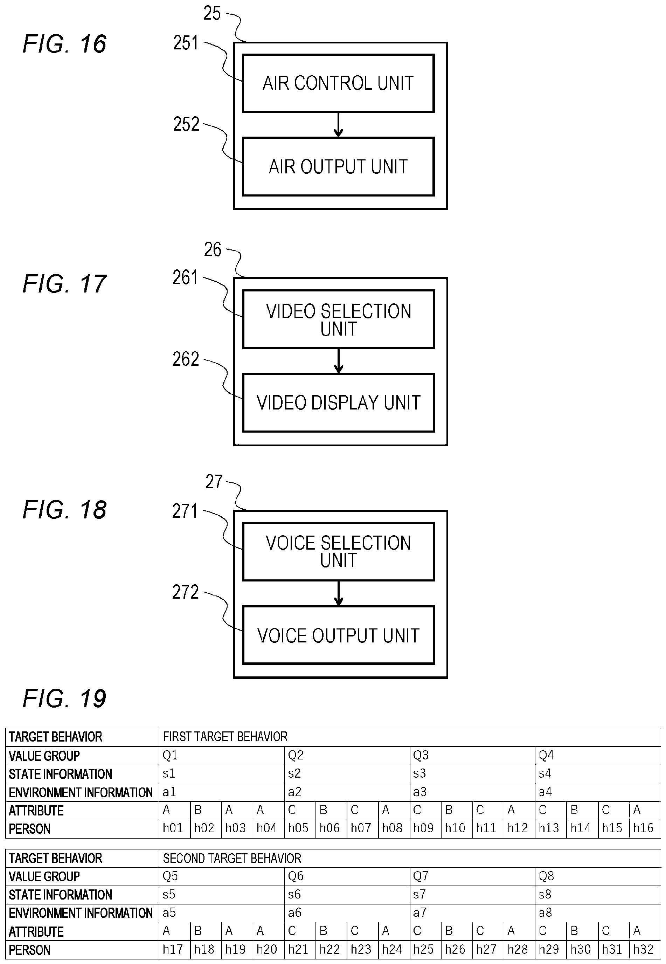

A configuration of the air conditioning unit 25 will be described with reference to Fig. 16. Fig. 16 is a block diagram illustrating a configuration of the air conditioning unit 25 according to the present embodiment. As illustrated in Fig. 16, the air conditioning unit 25 can include, for example, an air control unit 251 and an air output unit 252.

The air control unit 251 can determine a temperature and/or humidity of the air. The temperature and/or the humidity is determined according to the environment information selected by the machine learning classifier 14. For example, the temperature may be determined to be 25.5 to 27.5°C, and the humidity may be determined to be 45 to 50%.

The air output unit 252 outputs an air on the basis of the information determined by the air control unit 251.

[(6) Video Control Unit]

The environmental control device 20 can include, for example, the video control unit 26 to control a video displayed to a person. The video control unit 26 controls a video to be displayed on the basis of the environment information selected by the machine learning classifier 14. The environmental control device 20 including the video control unit 26 can be implemented using, for example, a television, a portable game machine, a PC, a tablet, a smartphone, a head mounted display (HMD), a wearable device or a car navigation system.

Note that the video includes both moving and still images. Furthermore, the video may include a sound.

The person is prompted to engage in the target behavior unconsciously by visually recognizing the video displayed by the video control unit 26 and optimized for them.

A configuration of the video control unit 26 will be described with reference to Fig. 17. Fig. 17 is a block diagram illustrating a configuration of the video control unit 26 according to the present embodiment. As illustrated in Fig. 17, the video control unit 26 can include, for example, a video selection unit 261 and a video display unit 262.

The video selection unit 261 selects a video to be output. A selection process is not particularly limited, but for example, the video selection unit 261 can determine using, for example, an address at which a video file is recorded or a code of an advertisement banner. The address or the code is determined according to the environment information selected by the machine learning classifier 14. Further, the video selection unit 261 may synthesize or edit a plurality of video files. Moreover, the video selection unit 261 may adjust, for example, a color temperature or luminance of the video.

In addition, the video file may be recorded in the video control unit 26 or may be recorded outside the video control unit 26.

The video display unit 262 outputs a video on the basis of the information determined by the video selection unit 261.

[(7) Sound Control Unit]

The environmental control device 20 can include, for example, the sound control unit 27 to control a sound played for a person. The sound control unit 27 controls a sound to be played on the basis of the environment information selected by the machine learning classifier 14. The environmental control device 20 including the sound control unit 27 can be implemented using, for example, a speaker (including a so-called smart speaker and a speaker with a streaming function), a tablet device, a smartphone, a headphone, a wearable device or a car stereo.

The person is prompted to engage in the target behavior unconsciously by listening to the sound played by the sound control unit 27 and optimized for them.

A configuration of the sound control unit 27 will be described with reference to Fig. 18. Fig. 18 is a block diagram illustrating a configuration of the sound control unit 27 according to the present embodiment. As illustrated in Fig. 18, the sound control unit 27 can include, for example, a sound selection unit 271 and a sound output unit 272.

The sound selection unit 271 selects a sound to be played. A selection process is not particularly limited, but for example, the sound selection unit 271 can determine using, for example, an address at which an audio file is recorded or a code of an advertisement banner. The address or the code is determined according to the environment information selected by the machine learning classifier 14. Further, the sound selection unit 271 may synthesize or edit a plurality of audio files. Furthermore, the sound selection unit 271 may adjust, for example, a pitch and a volume.

Moreover, the audio file may be recorded in the sound control unit 27 or may be recorded outside the sound control unit 27.

The sound output unit 272 outputs a sound on the basis of the information determined by the sound selection unit 271.

[3. Third Embodiment of the Present Technology (Example 3 of Machine Learning System)]

The machine learning device 10 according to one embodiment of the present technology can record a value function Q, a state information s, and a change a of the environment information for each target behavior. Then, the machine learning device 10 can then select the environment information that can prompt a person to engaging in the target behavior by performing reinforcement learning on the correlation between the person's behavior and the environment around the person.

At this time, a plurality of persons having a similar correlation between a behavior and an environment can be put in the same value group. For example, a plurality of persons who are likely to be prompted to engage in a specific target behavior when feeling a scent and a temperature change can be put in the same group.

This will be described with reference to Fig. 19. Fig. 19 illustrates one example of a database used by the machine learning device 10 according to the present embodiment. As illustrated in Fig. 19, the database holds the value function Q, the state information s, and the change a in the environment information for each target behavior. The value function Q is divided into a plurality of value groups Q1 to Q8 according to a similarity level of a correlation between the state information s and the change a in the environment information. Persons h01 to h32 are respectively belonging to each of the plurality of value groups Q1 to Q8 and associated with each value group. Attribute information A to C indicating features of each person may be associated with each person.

When a value group to which a subject of reinforcement learning belongs is known, the machine learning device 10 can use information such as the value function Q related to this value group. By using information such as the value function Q that has already undergone reinforcement learning, for example, the machine learning device 10 can partially omit a reinforcement learning process and reduce a time taken to perform reinforcement learning.

A specific example will be described hereinbelow. It is assumed that a target behavior for a certain person is set to "play a video game". It is also assumed that it is found by reinforcement learning that they tend to play a video game when affected by a scent and a temperature change.

Next, the target behavior is changed from "play a video game" to "drink beer". The previous reinforcement learning shows that this person is easily affected by a scent and a temperature change. Consequently, information such as the value function Q of the value group susceptible to a scent and a temperature change and the change a of the environment information for which the higher reward has been obtained can be used as initial values of reinforcement learning for prompting the person to engage in the new target behavior. The machine learning device 10 can initiate reinforcement learning using the information that has already been experienced reinforcement learning as the initial value.

Further, the information such as the value function Q that has been already experienced reinforcement learning may be used for reinforcement learning on a behavior of another person belonging to the same value group. Referring to Fig. 19, for example, information such as the value function Q that has been already experienced reinforcement learning on a behavior of a person h13 belonging to a value group Q4 can be used for reinforcement learning on a behavior of a person h14 belonging to the same value group Q4.

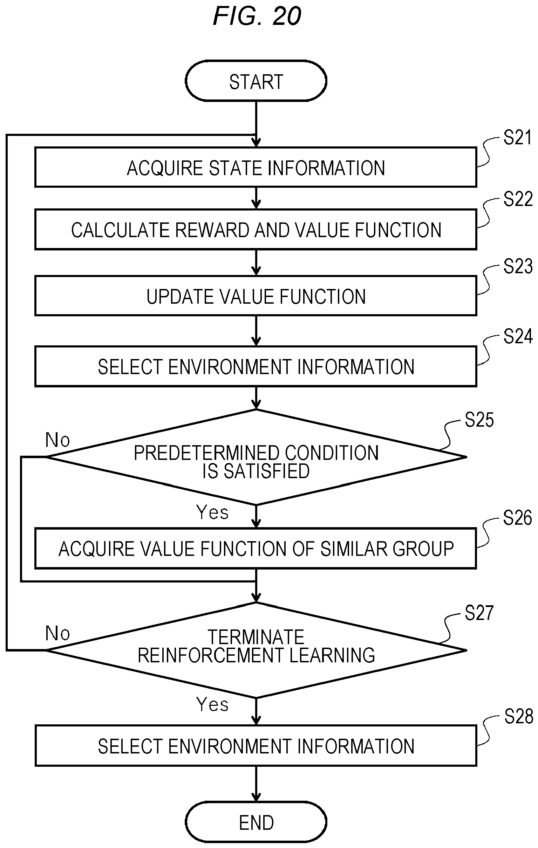

A procedure of the machine learning device 10 according to the present embodiment will be described referring to Fig. 20. Fig. 20 is a flowchart illustrating an exemplified procedure of the machine learning device 10 according to the present embodiment.

As illustrated in Fig. 20, the state acquisition unit 11 included in the machine learning device 10 acquires the state information (step S21).

Next, the evaluation unit 12 included in the machine learning device 10 calculates a reward and a value function on the basis of the state information (step S22).

Next, the machine learning classifier 14 included in the machine learning device 10 updates the value function (step S23).

Next, for learning further behavior modification, the machine learning classifier 14 selects the environment information (step S24).

Next, the machine learning classifier 14 determines whether or not a predetermined condition is satisfied (step S25). This determination condition is not particularly limited, but may be determined by, for example, whether or not the number of times of updating of the value function exceeds a predetermined threshold.

When the predetermined condition is satisfied (step S25: YES), the machine learning classifier 14 refers to the database and acquires information such as the value function Q of the similar group and the change a of the environment information in which a higher reward has been obtained (step S26). This database may be included in the machine learning device 10 or may be included in a computer device other than the machine learning device 10. The machine learning device 10 can perform reinforcement learning using the information that has already been experienced reinforcement learning.

On the other hand, when the predetermined condition is not satisfied (step S25: NO), the value function of the similar group is not acquired.

Next, the machine learning classifier 14 determines whether or not reinforcement learning should be terminated (step S27). This determination condition is not particularly limited, but may be determined by, for example, whether or not the value function is greater than a predetermined threshold.

When it is determined that the machine learning should not be terminated (step S27: NO), the procedure of steps S21 to S26 is repeated.

When it is determined that the machine learning should be terminated (step S27: YES), the machine learning classifier 14 selects the environment information (step S28).

[4. Fourth Embodiment of the Present Technology (Example 4 of Machine Learning System)]

The target behavior may be randomly set. By prompting various target behaviors without being limited to a specific target behavior, the machine learning classifier 14 can perform reinforcement learning of a correlation between a behavior and an environment. With the reinforcement learning, the machine learning classifier 14 can find regularity such as signs of a behavior and continuity, for example, even in a change in an environment that is considered to have a low relationship with a behavior.

This will be described also with reference to Fig. 19 Each of the plurality of persons enrolled in the database is associated with attribute information indicating features of that person. For example, an attribute A may be a person having features that "tend to drink beer when a color temperature of a lighting is 3650 K, a luminance of the lighting is 3000 lm, and a temperature is 26.5°C". For example, an attribute C may be a person having features that "tend to access e-commerce websites when a temperature is 25°C, a humidity is 48%, and a scent contains an additive T".

To implement this scheme stated above, a value function of a randomly selected group may be acquired, instead of acquiring a value function of a similar group (step S26), as shown in the flowchart illustrated in Fig. 20.

[5. Fifth Embodiment of the Present Technology (Example 5 of Machine Learning System)]

[(1) Overview]

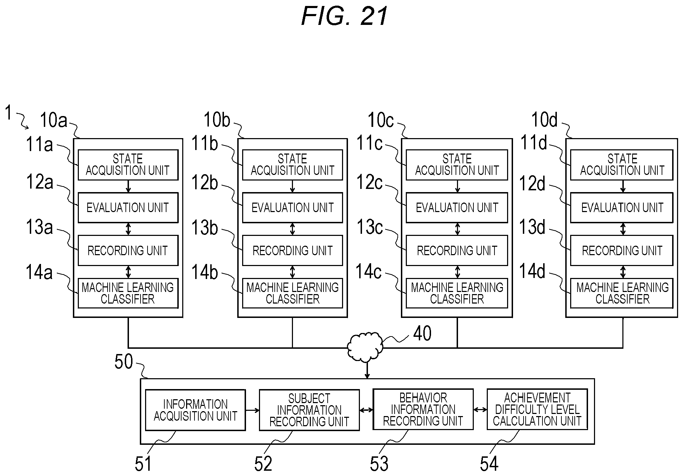

The machine learning system 1 according to one embodiment of the present technology may include a plurality of machine learning devices. This will be described with reference to Fig. 21. Fig. 21 is a block diagram illustrating a configuration of the machine learning system 1 according to one embodiment of the present technology.

As illustrated in Fig. 21, the machine learning system 1 can include, for example, a plurality of machine learning devices 10a to 10d. Each of the plurality of machine learning devices 10a to 10d can respectively include, for example, state acquisition units 11a to 11d, evaluation units 12a to 12d, recording units 13a to 13d, and machine learning classifiers 14a to 14d. Furthermore, an environmental control device (not illustrated) may be connected to each of the plurality of machine learning devices 10a to 10d. Moreover, the number of machine learning devices is not particularly limited.

Furthermore, the machine learning system 1 can include an achievement difficulty level calculation device 50. The achievement difficulty level calculation device 50 may have a hardware configuration as illustrated in Fig. 10. The achievement difficulty level calculation device 50 is connected to each of the plurality of machine learning devices 10a to 10d via, for example, the information communication network 40, and can aggregate information obtained from each of the plurality of machine learning devices 10a to 10d, whereby obtaining a tendency of a correlation between the state information and the environment information. More specifically, the achievement difficulty level calculation device 50 can calculate an achievement difficulty level for the target behavior. When it is defined that the prompted target behavior is successfully achieved, the achievement difficulty level indicates how difficult this achievement is. The achievement difficulty level will be described in detail later.

The achievement difficulty level calculation device 50 can include, for example, an information acquisition unit 51, a subject information recording unit 52, a behavior information recording unit 53, and an achievement difficulty level calculation unit 54.

The information acquisition unit 51 acquires the state information obtained by each of the plurality of machine learning devices 10a to 10d. The information acquisition unit 51 can be implemented using, for example, the communication interface 104.

Each of the plurality of machine learning devices 10a to 10d may target a different subject. The subject information recording unit 52 holds information regarding a subject targeted by each of the plurality of machine learning devices 10a to 10d. This information includes, for example, an identification number, gender or age of the subject. The subject information recording unit 52 can be implemented using, for example, the storage 102.

The behavior information recording unit 53 holds information regarding a target behavior set for each of the plurality of machine learning devices 10a to 10d. This information includes, for example, information regarding the target behavior, state information, and history information regarding the state information. The behavior information recording unit 53 can be implemented using, for example, the storage 102.

The achievement difficulty level calculation unit 54 can calculate an achievement difficulty level for the target behavior on the basis of the state information acquired by each of a plurality of state acquisition units 11a to 11d. The achievement difficulty level calculation unit 54 can be implemented using, for example, the CPU 101 and a program.

Further, the achievement difficulty level calculation unit 54 may be included in the achievement difficulty level calculation device 50, may be included in each of the plurality of machine learning devices 10a to 10d, or may be included in each of the plurality of environmental control devices (not shown).

Moreover, although not illustrated, the machine learning system 1 can include a plurality of achievement difficulty level calculation devices. Among the plurality of achievement difficulty level calculation devices, there may be an achievement difficulty level calculation device for relay, which aggregates information obtained from a specific machine learning device among the plurality of machine learning devices.

[(2) Achievement Difficulty Level]

As described above, the achievement difficulty level indicates the difficulty in prompting the target behavior. By calculating the achievement difficulty level, for example, the machine learning system 1 can derive a subject who is likely or less likely to be prompted to engage in a target behavior, or derive environment information in which a subject is likely or less likely to be prompted to engage in a target behavior.

A group of subjects who are likely to be prompted to engage in a target behavior is defined as an adaptive group, and a group of subjects who are less likely to be prompted to engage in a target behavior is defined as a challenge group. The machine learning system 1 can derive a target audience of a product, for example, in product development or advertisement promotion, by deriving the adaptive group. The target audience includes, for example, age and gender. Product development and advertisement promotion can be carried out more efficient by deriving the target audient of a product.

For example, a point-of-sale (POS) system provided in, for example, a convenience store can be associated with a local event (e.g. sports or firework festival). Consequently, the machine learning system 1 can derive, for example, a product that is likely to be purchased during the event and a target audience of the product.

Alternatively, examples of the product include hot-selling and long-selling products. The adaptive group can be utilized for the development and advertisement activities for the former, and the challenge group can be utilized for the development and advertisement activities of the latter.

Furthermore, the target behaviors can be classified into a basic target behavior and an applied target behavior associated with the basic target behavior. The basic target behavior includes behaviors that are roughly classified by type, for example, "going out", "eating and drinking" and "purchasing". The applied target behavior more specifically indicates the basic target behavior; for example, "going to a specific shop on Black Friday", "going to a specific place" and "participating in a local festival".

The machine learning system 1 first derives an adaptive group that is likely to be prompted to engage in the applied target behavior. The machine learning system 1 can derive an adaptive group related to the basic target behavior by deriving the adaptive group related to each of a plurality of applied target behaviors and appealing information regarding the adaptive group. That is, the machine learning system 1 can obtain a tendency common to a plurality of adaptive groups. Consequently, for example, a new target audience that has not been noticed until now can be derived for product development.

Further, derivation of the adaptive group may also be used to improve daily habits as described in the second embodiment.

The achievement difficulty level may include, for example, an achievement rate r indicating a degree to which the target behavior is prompted. Subjects with a higher achievement rate r are classified into the adaptive group.

The achievement rate r can be represented by, for example, the following Equation (3) using the number n of pieces of state information that the target behavior is prompted and the number $n_{all}$ of all pieces of state information including state information that the target behavior is not prompted.

[Math. 3]

$$r = \frac{100n}{n_{all}} \quad \cdots \quad (3)$$

The achievement difficulty level may include, for example, a standard achievement time s indicating a standard time for which the target behavior is prompted. Subjects with a shorter standard achievement time s are classified into the adaptive group.

The standard achievement time s can be represented by, for example, the following Equation (4) using an achievement time x indicating a time taken to prompt a subject to engage in the target behavior and an average achievement time p indicating an average time taken to prompt a subject to engage in the target behavior. The average achievement time p can be calculated by dividing the sum of the achievement times x by the number $n_{all}$ of all pieces of state information.

[Math. 4]

$$s = \sqrt{\frac{1}{n}\sum_{i=1}^{n}(x_i - p)^2} \quad \cdots \quad (4)$$

Further, although the standard achievement time s is calculated using the standard deviation so as not to be affected by a subject having an extremely long achievement time, the average achievement time p using the average instead of the standard deviation may be included in the achievement difficulty level.

Alternatively, the achievement difficulty level may include, for example, the number q of key variables indicating an average number of items in the environment information when the target behavior is prompted. Examples of the items in the environment information include scent, lighting, temperature, humidity, video or sound. Subjects with a smaller number q of key variables are classified into the adaptive group. For example, a subject who is only affected by a scent is more likely to be prompted to engage in a target behavior than a subject who is not affected by both a scent and a temperature.

The number q of key variables can be represented by, for example, the following Equation (5) using the number n of pieces of state information that the target behavior is prompted and the number e of items in the environment information when the target behavior is prompted. Further, the achievement difficulty level calculation unit 54 may calculate the standard deviation instead of the average, as in Equation (4).

[Math. 5]

$$q = \frac{1}{n}\sum_{i=1}^{n} e_i \quad \cdots \quad (5)$$

Names of the items in the environment information may be recorded together with the calculation of the number q of key variables. For example, the behavior information recording unit 53 can record the names of the items in the environment information. Consequently, the machine learning system 1 can derive an adaptive group in which behavior modification is easily prompted for specific environment information. For example, the machine learning system 1 can derive an adaptive group in which behavior modification is easily prompted for a scent.

Further, the achievement difficulty level may include at least one of the achievement rate r, the standard achievement time s and the number q of key variables. However, for example, the adaptive group can be more easily derived in a case where both the achievement rate r and the standard achievement time s are included in the achievement difficulty level than in a case where only the achievement rate r is included in the achievement difficulty level.

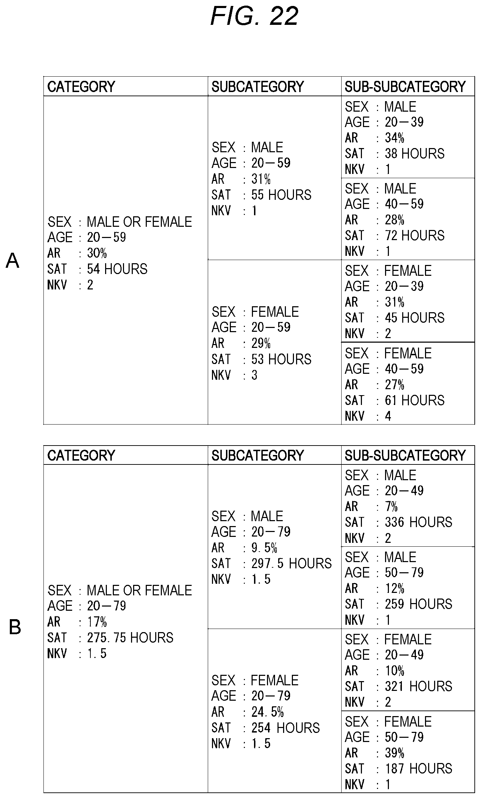

The achievement difficulty level will be described with reference to Fig. 22. Fig. 22 is a diagram illustrating the achievement difficulty level calculated by the achievement difficulty level calculation unit 54 according to the present embodiment. As illustrated in Fig. 22, the achievement rate (AR), the standard achievement time (SAT), and the number of key variables (NKV) included in the achievement difficulty level are represented.

Fig. 22A illustrates an achievement difficulty level when a target behavior "take exercise" is prompted to subjects who do not take exercise every day. As shown in a category, subjects who are prompted to engage in this target behavior are men and women of 20 to 59 years. The achievement rate of all subjects is 30%, the standard achievement time is 54 hours, and the number of key variables is 2.

Subcategory is a subdivision of the category. The category herein is subdivided on the basis of gender as an example. The achievement rate of all males is 310, the standard achievement time is 55 hours, and the number of key variables is 1. On the other hand, the achievement rate of all females is 29%, the standard achievement time is 53 hours, and the number of key variables is 3. This shows that a male subject has a higher achievement rate than a female subject. In other words, male subjects correspond to the adaptive group when focusing on the achievement rate.

Sub-subcategory is a subdivision of the subcategory. The subcategory herein is subdivided on the basis of age as an example. The achievement rate of all males of 20 to 39 years is 34%, the standard achievement time is 38 hours, and the number of key variables is 1. Among four groups under this sub-subcategory, this group has the highest achievement rate, the shortest standard achievement time, and the smallest number of key variables. That is, this group corresponds to the adaptive group. The machine learning system 1 can derive the adaptive group in this manner. Promotional activities about products and services related to the target behavior "take exercise" can be directed towards this adaptive group.

Fig. 22B shows an achievement difficulty level when a target behavior "go vote" is prompted to subjects who have never voted in the past 5 years. Among four groups under a sub-subcategory, a group of female subjects of 50 to 79 years has the highest achievement rate, the shortest standard achievement time, and the smallest number of key variables. That is, this group corresponds to the adaptive group.

As described above, the machine learning system 1 can derive an adaptive group by calculating the achievement rate, the standard achievement time or the number of key variables. For example, the machine learning system 1 can derive an adaptive group having the achievement rate ≥ 80% and the standard achievement time ≤ 3 hours for a target behavior "buy beer". A beer company can make advertisement and promotional activities of a new product focusing on the adaptive group when they launch the new product.

Further, for example, the machine learning system 1 can derive an adaptive group having the achievement rate ≥ 90% and the number of key variables ≤ 2 for a target behavior "watch TV shows or videos at online streaming platform". A video streaming service provider may make advertisement and promotional activities of subscription to their service focusing on the adaptive group. The service provider may make advertisement and promotional activities of encouraging renewal focusing on the adaptive group even after they have become subscribers.

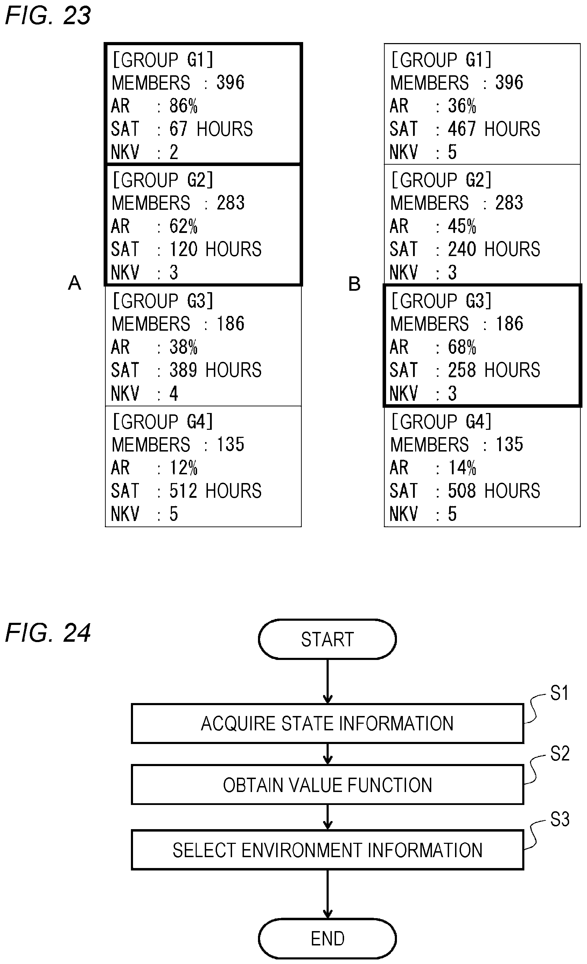

A value of this achievement difficulty level may alter as the target behavior is repeatedly prompted. This will be described with reference to Fig. 23. Fig. 23 is a diagram illustrating the achievement difficulty level calculated by the achievement difficulty level calculation unit 54 according to the present embodiment.

Fig. 23A illustrates an achievement difficulty level when a target behavior "buy product S at least twice" is prompted. Further, in this case, the achievement rate is 100% when the product S is bought twice, and 50% when the product S is purchased once.

In Fig. 23A, subjects are classified into a plurality of groups on the basis of the achievement difficulty level. For example, a first group G1 has 396 subjects, in which the achievement rate is 86%, the standard achievement time is 67 hours, and the number of key variables is 2. Moreover, a second group G2 has 283 subjects, in which the achievement rate is 62%, the standard achievement time is 120 hours, and the number of key variables is 3. Among four groups, the first group G1 and the second group G2 have the higher achievement rate, the shorter standard achievement time, and the smaller number of key variables. That is, the first group G1 and the second group G2 correspond to the adaptive group. A seller of the product S can prompt the adaptive group to engage in a target behavior related to the product S.

Subsequently, the machine learning system 1 prompts the first group G1 and the second group G2 to engage in the target behavior related to the product S, and prompts a third group G3 and a fourth group G4, which correspond to a challenge group, to engage in a target behavior related to a product T, which is another product.

Fig. 23B illustrates an achievement difficulty level when a target behavior "buy product T at least twice" is prompted. As illustrated in Fig. 23B, a third group G3 has the achievement rate of 68%, the standard achievement time of 258 hours, and the number of key variables of 3. Among four groups, the third group G3 has the higher achievement rate, the shorter standard achievement time, and the smaller number of key variables. That is, the third group G3 corresponds to the adaptive group.

That is, by setting the first group G1 and the second group G2 a target audience of the product S, and setting the third group G3 as a target audience of the product T, for example, the sales of the product or efficiency of the promotional activities can be improved.

[6. Sixth Embodiment of the Present Technology (Machine Learning Method)]

A machine learning method according to one embodiment of the present technology is a machine learning method for training a correlation between a person's behavior and an environment around the person using a computer device. The machine learning method according to the present embodiment will be described referring to Fig. 24. Fig. 24 is a flowchart illustrating a procedure of the machine learning method according to the present embodiment. As illustrated in Fig. 24, the machine learning method according to the present embodiment includes at least: acquiring at least state information regarding a behavior of a person (step S1); obtaining a value function by evaluating the state information and environment information regarding an environment around the person when acquiring the state information (step S2); and performing reinforcement learning on the value function and selecting the environment information when the value function is the highest in order to prompt the person to engage in a target behavior (step S3).

The machine learning method according to the present embodiment may use the technology according to the first to fourth embodiments. Therefore, the descriptions will be omitted.

Further, advantageous effects described in the present specification are merely examples and are not limited, and other effects may be expected.

Furthermore, the present technology can also have the following configurations.

[1] A machine learning system, including at least:

a state acquisition unit configured to acquire at least state information regarding a behavior of a person;
an evaluation unit configured to obtain a value function by evaluating the state information and environment information regarding an environment around the person when acquiring the state information; and
a machine learning classifier that performs reinforcement learning on the value function and selects the environment information when the value function is the highest in order to prompt the person to engage in a target behavior.

[2] The machine learning system as set forth in [1],

in which the evaluation unit is configured
to calculate a reward on the basis of a difference between the state information and target state information regarding the target behavior, and
to calculate the value function on the basis of the reward, the environment information and the state information.

[3] The machine learning system as set forth in [1] or [2],
in which the system holds target state-related information including a plurality of pieces of target behavior information.
[4] The machine learning system as set forth in [3],
in which the target state-related information includes time-specific target state information and/or stage-specific target state information.
[5] The machine learning system as set forth in any one of [1] to [4],
in which the environment information includes information regarding scents, lighting, temperature, humidity, video or sound.
[6] The machine learning system as set forth in any one of [1] to [5],

further including a scent control unit,
in which the scent control unit is configured to control generated scent on the basis of the environment information selected by the machine learning classifier.

[7] The machine learning system as set forth in [6],

further including an aromatization unit,
in which the aromatization unit is configured to make items have scent on the basis of the environment information selected by the machine learning classifier, and
the machine learning classifier determines which of the scent control unit and the aromatization unit will generate scent on the basis of the environment information.

[8] The machine learning system as set forth in any one of [1] to [7],

further including a lighting control unit,
in which the lighting control unit is configured to control light to be emitted on the basis of the environment information selected by the machine learning classifier.

[9] The machine learning system as set forth in any one of [1] to [8],

further including an air conditioning unit,
in which the air conditioning unit is configured to control a temperature and/or humidity on the basis of the environment information selected by the machine learning classifier.

[10] The machine learning system as set forth in any one of [1] to [9],

further including a video control unit,
in which the video control unit is configured to control a video to be displayed on the basis of the environment information selected by the machine learning classifier.

[11] The machine learning system as set forth in any one of [1] to [10],

further including a sound control unit,
in which the sound control unit is configured to control a sound to be played on the basis of the environment information selected by the machine learning classifier.

[12] The machine learning system as set forth in any one of [1] to [11],

in which the value function is divided into a plurality of value groups, and
the machine learning classifier uses the value function held by each of the plurality of value groups.

[13] The machine learning system as set forth in any one of [1] to [12], further including:

a plurality of state acquisition units; and
an achievement difficulty level calculation unit,
in which the achievement difficulty level calculation unit is configured to calculate an achievement difficulty level for the target behavior on the basis of the state information acquired by each of the plurality of state acquisition units.

[14] The machine learning system as set forth in [13],
in which the achievement difficulty level includes an achievement rate indicating a degree to which the target behavior is prompted.
[15] The machine learning system as set forth in [13] or [14],
in which the achievement difficulty level includes a standard achievement time indicating a standard time for which the target behavior is prompted.
[16] The machine learning system as set forth in any one of [13] to [15],
in which the achievement difficulty level includes a number of key variables indicating an average number of items in the environment information when the target behavior is prompted.
[17] A machine learning device, including at least:

a state acquisition unit configured to acquire at least state information regarding a behavior of a person;
an evaluation unit configured to obtain a value function by evaluating the state information and environment information regarding an environment around the person when acquiring the state information; and
a machine learning classifier that performs reinforcement learning on the value function and selects the environment information when the value function is the highest in order to prompt the person to engage in a target behavior.

[18] A machine learning method, including at least:

acquiring at least state information regarding a behavior of a person;
obtaining a value function by evaluating the state information and environment information regarding an environment around the person when acquiring the state information; and
performing reinforcement learning on the value function and selecting the environment information when the value function is the highest in order to prompt the person to engage in a target behavior.

REFERENCE SIGNS LIST

1    Machine learning system

10    Machine learning device
11    State acquisition unit
12    Evaluation unit
13    Recording unit
14    Machine learning classifier
20    Environmental control device
23    Scent control unit
24    Lighting control unit
25    Air conditioning unit
26    Video control unit
27    Sound control unit
30    Aromatization unit
40    Information communication network
50    Achievement difficulty level calculation device
51    Information acquisition unit
52    Subject information recording unit
53    Behavior information recording unit
54    Achievement difficulty level calculation unit
51    Acquire at least state information
S2    Obtain value function by evaluating environment information and state information
S3    Select environment information

**Claims**

1. A machine learning system, comprising at least:

   a state acquisition unit configured to acquire at least state information regarding a behavior of a person;
   an evaluation unit configured to obtain a value function by evaluating the state information and environment information regarding an environment around the person when acquiring the state information; and
   a machine learning classifier that performs reinforcement learning on the value function and selects the environment information when the value function is the highest in order to prompt the person to engage in a target behavior.

2. The machine learning system according to claim 1,

   wherein the evaluation unit is configured
   to calculate a reward on a basis of a difference between the state information and target state information regarding the target behavior, and
   to calculate the value function on a basis of the reward, the environment information and the state information.

3. The machine learning system according to claim 1,
   wherein the system holds target state-related information including a plurality of pieces of target behavior information.

4. The machine learning system according to claim 3,
   wherein the target state-related information includes time-specific target state information and/or stage-specific target state information.

5. The machine learning system according to claim 1,
   wherein the environment information includes information regarding scents, lighting, temperature, humidity, video or sound.

6. The machine learning system according to claim 1,

   further comprising a scent control unit,
   wherein the scent control unit is configured to control generated scent on a basis of the environment information selected by the machine learning classifier.

**7.** The machine learning system according to claim 6,

further comprising an aromatization unit,
wherein the aromatization unit is configured to make items have scent on a basis of the environment information selected by the machine learning classifier, and
the machine learning classifier determines which of the scent control unit and the aromatization unit will generate scent on a basis of the environment information.

**8.** The machine learning system according to claim 1,

further comprising a lighting control unit,
wherein the lighting control unit is configured to control light to be emitted on a basis of the environment information selected by the machine learning classifier.

**9.** The machine learning system according to claim 1,

further comprising an air conditioning unit,
wherein the air conditioning unit is configured to control a temperature and/or humidity on a basis of the environment information selected by the machine learning classifier.

**10.** The machine learning system according to claim 1,

further comprising a video control unit,
wherein the video control unit is configured to control a video to be displayed on a basis of the environment information selected by the machine learning classifier.

**11.** The machine learning system according to claim 1,

further comprising a sound control unit,
wherein the sound control unit is configured to control a sound to be played on a basis of the environment information selected by the machine learning classifier.

**12.** The machine learning system according to claim 1,

wherein the value function is divided into a plurality of value groups, and
the machine learning classifier uses the value function held by each of the plurality of value groups.

**13.** The machine learning system according to claim 1, further comprising:

a plurality of state acquisition units; and
an achievement difficulty level calculation unit,
wherein the achievement difficulty level calculation unit is configured to calculate an achievement difficulty level for the target behavior on a basis of the state information acquired by each of the plurality of state acquisition units.

**14.** The machine learning system according to claim 13,
wherein the achievement difficulty level includes an achievement rate indicating a degree to which the target behavior is prompted.

**15.** The machine learning system according to claim 13,
wherein the achievement difficulty level includes a standard achievement time indicating a standard time for which the target behavior is prompted.

**16.** The machine learning system according to claim 13,
wherein the achievement difficulty level includes a number of key variables indicating an average number of items in the environment information when the target behavior is prompted.

**17.** A machine learning device, comprising at least:

a state acquisition unit configured to acquire at least state information regarding a behavior of a person;

an evaluation unit configured to obtain a value function by evaluating the state information and environment information regarding an environment around the person when acquiring the state information; and

a machine learning classifier that performs reinforcement learning on the value function and selects the environment information when the value function is the highest in order to prompt the person to engage in a target behavior.

18. A machine learning method, comprising at least:

acquiring at least state information regarding a behavior of a person;

obtaining a value function by evaluating the state information and environment information regarding an environment around the person when acquiring the state information; and

performing reinforcement learning on the value function and selecting the environment information when the value function is the highest in order to prompt the person to engage in a target behavior.

FIG. 1

FIG. 2

FIG. 3

## FIG. 4

THIRD LEVEL BEHAVIOR

SECOND LEVEL BEHAVIOR — WATCHED CONTENTS | READ A BOOK | STARTED COOKING | DID SOME STRETCHES

FIRST LEVEL BEHAVIOR — LOOKED AWAY | HANDS ARE IDLE

TARGET BEHAVIOR — ACCESS TO SNS ACCOUNTS

## FIG. 5

THIRD LEVEL BEHAVIOR

SECOND LEVEL BEHAVIOR — CUT HAIR | WENT HOME | LEFT THE ROOM | ATE CHOCOLATE

FIRST LEVEL BEHAVIOR — SCROLLED DOWN A SCREEN | CHANGED ITEMS IN A SHOPPING CART

TARGET BEHAVIOR — MAKE A BIG PURCHASE

## FIG. 6

THIRD LEVEL BEHAVIOR

SECOND LEVEL BEHAVIOR — WATCHED TV | TOOK A BATH | WENT HOME | HAD A MEAL

FIRST LEVEL BEHAVIOR — OPENED A REFRIGERATOR | WATCHED TV

TARGET BEHAVIOR — DRINK BEER

## FIG. 7

THIRD LEVEL BEHAVIOR

SECOND LEVEL BEHAVIOR — WENT HOME | WATCHED TV | TOOK A BATH | DRANK ALCOHOL

FIRST LEVEL BEHAVIOR — USED A MOBILE PHONE | SAT ON A SOFA

TARGET BEHAVIOR — GO TO SLEEP

## FIG. 8

A

CUT HAIR  BOUGHT COSMETICS  WENT TO GYM  SAW A CALENDAR

LOOKED INTO A CLOSET  LOOKED INTO A MIRROR

BUY CLOTHES

B

DRANK COFFEE  PLAYED A GAME  DID HOUSEWORK  WALKED A PET

HAD SWEETS  HAD JUNK FOODS

BUY CLOTHES

## FIG. 9

| TIME ZONE | TARGET BEHAVIOR |
|---|---|
| FIRST TIME ZONE (AROUND 12:00 AM—6:00 AM) | GO TO SLEEP |
| SECOND TIME ZONE (AROUND 7:00 AM—7:00 PM) | EAT FOOD S |
| THIRD TIME ZONE (AROUND 8:00 PM—11:00 PM) | DRINK BEVERAGE T |

## FIG. 10

10

101 CPU

103 RAM

102 STORAGE

104 COMMUNICATION INTERFACE

## FIG. 11

## FIG. 12

## FIG. 13

*FIG. 14*

START

PERFORM REINFORCEMENT LEARNING ON
CORRELATION BETWEEN SCENT AND BEHAVIOR ⟶ S11

SELECT ENVIRONMENT INFORMATION ⟶ S12

DETERMINE SCENT CONTROL UNIT ⟶ S13

CONTROL SCENT ⟶ S14

No ◁ FULLY LEARNED CORRELATION ▷ S15

Yes

DETERMINE AROMATIZATION UNIT ⟶ S16

MAKE ITEMS HAVE SCENT ⟶ S17

END

*FIG. 15*

24

241 — LIGHT CONTROL UNIT

242 — LIGHT OUTPUT UNIT

*FIG. 16*

AIR CONTROL UNIT

AIR OUTPUT UNIT

*FIG. 17*

VIDEO SELECTION UNIT

VIDEO DISPLAY UNIT

*FIG. 18*

VOICE SELECTION UNIT

VOICE OUTPUT UNIT

*FIG. 19*

| TARGET BEHAVIOR | FIRST TARGET BEHAVIOR | | | | | | | | | | | | | | | |
|---|---|---|---|---|---|---|---|---|---|---|---|---|---|---|---|---|
| VALUE GROUP | Q1 | | | | Q2 | | | | Q3 | | | | Q4 | | | |
| STATE INFORMATION | s1 | | | | s2 | | | | s3 | | | | s4 | | | |
| ENVIRONMENT INFORMATION | a1 | | | | a2 | | | | a3 | | | | a4 | | | |
| ATTRIBUTE | A | B | A | A | C | B | C | A | C | B | C | A | C | B | C | A |
| PERSON | h01 | h02 | h03 | h04 | h05 | h06 | h07 | h08 | h09 | h10 | h11 | h12 | h13 | h14 | h15 | h16 |

| TARGET BEHAVIOR | SECOND TARGET BEHAVIOR | | | | | | | | | | | | | | | |
|---|---|---|---|---|---|---|---|---|---|---|---|---|---|---|---|---|
| VALUE GROUP | Q5 | | | | Q6 | | | | Q7 | | | | Q8 | | | |
| STATE INFORMATION | s5 | | | | s6 | | | | s7 | | | | s8 | | | |
| ENVIRONMENT INFORMATION | a5 | | | | a6 | | | | a7 | | | | a8 | | | |
| ATTRIBUTE | A | B | A | A | C | B | C | A | C | B | C | A | C | B | C | A |
| PERSON | h17 | h18 | h19 | h20 | h21 | h22 | h23 | h24 | h25 | h26 | h27 | h28 | h29 | h30 | h31 | h32 |

## FIG. 20

```
                    START

                                                    S21
        ACQUIRE STATE INFORMATION

                                                    S22
    CALCULATE REWARD AND VALUE FUNCTION

                                                    S23
         UPDATE VALUE FUNCTION

                                                    S24
       SELECT ENVIRONMENT INFORMATION

                                                    S25
  No        PREDETERMINED CONDITION
                 IS SATISFIED

                      Yes                           S26
    ACQUIRE VALUE FUNCTION OF SIMILAR GROUP

                                                    S27
  No              TERMINATE
            REINFORCEMENT LEARNING

                      Yes                           S28
       SELECT ENVIRONMENT INFORMATION

                     END
```

# FIG. 21

## FIG. 22

| CATEGORY | SUBCATEGORY | SUB-SUBCATEGORY |
|---|---|---|
| SEX : MALE OR FEMALE<br>AGE : 20−59<br>AR   : 30%<br>SAT  : 54 HOURS<br>NKV  : 2 | SEX : MALE<br>AGE : 20−59<br>AR   : 31%<br>SAT  : 55 HOURS<br>NKV  : 1 | SEX : MALE<br>AGE : 20−39<br>AR   : 34%<br>SAT  : 38 HOURS<br>NKV  : 1 |
| | | SEX : MALE<br>AGE : 40−59<br>AR   : 28%<br>SAT  : 72 HOURS<br>NKV  : 1 |
| | SEX : FEMALE<br>AGE : 20−59<br>AR   : 29%<br>SAT  : 53 HOURS<br>NKV  : 3 | SEX : FEMALE<br>AGE : 20−39<br>AR   : 31%<br>SAT  : 45 HOURS<br>NKV  : 2 |
| | | SEX : FEMALE<br>AGE : 40−59<br>AR   : 27%<br>SAT  : 61 HOURS<br>NKV  : 4 |

A

| CATEGORY | SUBCATEGORY | SUB-SUBCATEGORY |
|---|---|---|
| SEX : MALE OR FEMALE<br>AGE : 20−79<br>AR   : 17%<br>SAT  : 275.75 HOURS<br>NKV  : 1.5 | SEX : MALE<br>AGE : 20−79<br>AR   : 9.5%<br>SAT  : 297.5 HOURS<br>NKV  : 1.5 | SEX : MALE<br>AGE : 20−49<br>AR   : 7%<br>SAT  : 336 HOURS<br>NKV  : 2 |
| | | SEX : MALE<br>AGE : 50−79<br>AR   : 12%<br>SAT  : 259 HOURS<br>NKV  : 1 |
| | SEX : FEMALE<br>AGE : 20−79<br>AR   : 24.5%<br>SAT  : 254 HOURS<br>NKV  : 1.5 | SEX : FEMALE<br>AGE : 20−49<br>AR   : 10%<br>SAT  : 321 HOURS<br>NKV  : 2 |
| | | SEX : FEMALE<br>AGE : 50−79<br>AR   : 39%<br>SAT  : 187 HOURS<br>NKV  : 1 |

B

*FIG. 23*

```
[GROUP G1]
MEMBERS : 396
AR      : 86%
SAT     : 67 HOURS
NKV     : 2

[GROUP G2]
MEMBERS : 283
AR      : 62%
SAT     : 120 HOURS
NKV     : 3

[GROUP G3]
MEMBERS : 186
AR      : 38%
SAT     : 389 HOURS
NKV     : 4

[GROUP G4]
MEMBERS : 135
AR      : 12%
SAT     : 512 HOURS
NKV     : 5
```
A

```
[GROUP G1]
MEMBERS : 396
AR      : 36%
SAT     : 467 HOURS
NKV     : 5

[GROUP G2]
MEMBERS : 283
AR      : 45%
SAT     : 240 HOURS
NKV     : 3

[GROUP G3]
MEMBERS : 186
AR      : 68%
SAT     : 258 HOURS
NKV     : 3

[GROUP G4]
MEMBERS : 135
AR      : 14%
SAT     : 508 HOURS
NKV     : 5
```
B

*FIG. 24*

START

ACQUIRE STATE INFORMATION — S1

OBTAIN VALUE FUNCTION — S2

SELECT ENVIRONMENT INFORMATION — S3

END

## INTERNATIONAL SEARCH REPORT

| | International application No. |
|---|---|
| | PCT/JP2021/001234 |

### A. CLASSIFICATION OF SUBJECT MATTER
Int. Cl. G06N20/00(2019.01)i
FI: G06N20/00

According to International Patent Classification (IPC) or to both national classification and IPC

### B. FIELDS SEARCHED

Minimum documentation searched (classification system followed by classification symbols)
Int. Cl. G06N3/00-99/00

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y<br>A | WO 2020/027174 A1 (DAINIPPON PRINTING CO., LTD.)<br>06 February 2020, paragraphs [0032]-[0057],<br>[0093]-[0102], [0112], [0134]-[0157], fig. 1, 2,<br>10, 15, 16 | 1-3, 5-6, 8-18<br>4, 7 |
| Y<br>A | JP 2019-28899 A (ASCON CO., LTD.) 21 February<br>2019, paragraphs [0018]-[0026], fig. 1 | 1-3, 5-6, 8-18<br>4, 7 |
| Y<br>A | WO 2019/087854 A1 (PANASONIC INTELLECTUAL PROPERTY<br>MANAGEMENT CO., LTD.) 09 May 2019, paragraphs<br>[0037]-[0042], [0100], [0101], fig. 3 | 1-3, 5-6, 8-18<br>4, 7 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 25.03.2021 | 06.04.2021 |

| Name and mailing address of the ISA/<br>　　Japan Patent Office<br>　　3-4-3, Kasumigaseki, Chiyoda-ku,<br>　　Tokyo 100-8915 Japan | Authorized officer<br><br>Telephone No. |

Form PCT/ISA/210 (second sheet) (January 2015)

**INTERNATIONAL SEARCH REPORT**

| International application No. |
| --- |
| PCT/JP2021/001234 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | 山崎惇広,外 1 名．逐次的に分割された問題空間における複素強化学習．電子情報通信学会論文誌．01 May 2011, vol. J94-D, no. 5, pp. 872-880, in particular, "5. Simulation Experiment", (YAMAZAKI, Atsuhiro et al. Complex-valued reinforcement learning in sequentially-partitioned problem space. IEICE Transactions on Information and Systems.) | 13-16 |

Form PCT/ISA/210 (continuation of second sheet) (January 2015)

**INTERNATIONAL SEARCH REPORT**
Information on patent family members

| | | International application No. |
|---|---|---|
| | | PCT/JP2021/001234 |

| Patent Documents referred to in the Report | Publication Date | Patent Family | Publication Date |
|---|---|---|---|
| WO 2020/027174 A1 | 06.02.2020 | (Family: none) | |
| JP 2019-28899 A | 21.02.2019 | (Family: none) | |
| WO 2019/087854 A1 | 09.05.2019 | CN 111316313 A | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019028899 A